# EUROPEAN PATENT APPLICATION

(11) **EP 3 936 128 A1**
(43) Date of publication of application: **12.01.2022**
(21) Application number: 20185333.0
(22) Date of filing: 10.07.2020
(51) Int. Cl.: A61K 31/44, A61K 31/4402, A61K 31/4406, A61K 31/4409, A61K 31/47, A61K 31/4965, A61K 31/50, C07D 221/00, A61P 33/02, A61P 33/06

(54) **ACTIVE SUBSTANCE AGAINST PROTOZOA**

(71) Applicant: Christian-Albrechts-Universität zu Kiel, 24118 Kiel (DE)
(72) Inventor: BEITZ, Eric, 24229 Danischenhagen (DE); WALLOCH, Philipp, 24118 Kiel (DE); HENKE, Björn, 24116 Kiel (DE)
(74) Representative: Taruttis, Stefan Georg

(57) **Abstract**

The present invention relates to active substances against protozoa, especially against Plasmodium spp., e.g. against Plasmodium falciparum, Plasmodium vivax, P. malariae, P. ovale , P. knowlesi, against Toxoplasma spp., e.g. Toxoplasma gondii, against Entamoeba histolytica, against Naegleria fowleri, and relates to a method of treatment and/or prophylaxis of an infection by a protozoon. The active substances are suitable for use in the treatment of and/or for use in prophylaxis of an infection by a protozoon. The active substances have the advantage of having activity against wild-type protozoa, especially against Plasmodium spp. and against Toxoplasma spp. and against Entamoeba histolytica and against Naegleria fowleri, and of having activity also against a mutant of P. falciparum that has been identified as resistant against other anti-malarial active substances.

SEQUENCE LISTING

<110> Christian-Albrechts-Universität zu Kiel

<120> Active substance against protozoa

<130> K1042EP

<160> 12

<170> BiSSAP 1.3.6

<210> 1
<211> 309
<212> PRT
<213> Plasmodium falciparum


<220>
<223> PfFNT_PF3D7_0316600_PlasmoDB

<400> 1
Met Pro Pro Asn Asn Ser Lys Tyr Val Leu Asp Pro Val Ser Ile Lys
1               5                   10                  15
Ser Val Cys Gly Gly Glu Glu Ser Tyr Ile Arg Cys Val Glu Tyr Gly
            20                  25                  30
Lys Lys Lys Ala His Tyr Ser Asn Leu Asn Leu Leu Ala Lys Ala Ile
        35                  40                  45
Leu Ala Gly Met Phe Val Gly Leu Cys Ala His Ala Ser Gly Ile Ala
    50                  55                  60
Gly Gly Leu Phe Tyr Tyr His Lys Leu Arg Glu Ile Val Gly Ala Ser
65                  70                  75                  80
Met Ser Val Phe Val Tyr Gly Phe Thr Phe Pro Ile Ala Phe Met Cys
                85                  90                  95
Ile Ile Cys Thr Gly Ser Asp Leu Phe Thr Gly Asn Thr Leu Ala Val
            100                 105                 110
Thr Met Ala Leu Tyr Glu Lys Lys Val Lys Leu Leu Asp Tyr Leu Arg
        115                 120                 125
Val Met Thr Ile Ser Leu Phe Gly Asn Tyr Val Gly Ala Val Ser Phe
    130                 135                 140
Ala Phe Phe Val Ser Tyr Leu Ser Gly Ala Phe Thr Asn Val His Ala
145                 150                 155                 160
Val Glu Lys Asn His Phe Phe Gln Phe Leu Asn Asp Ile Ala Glu Lys
                165                 170                 175
Lys Val His His Thr Phe Val Glu Cys Val Ser Leu Ala Val Gly Cys
            180                 185                 190
Asn Ile Phe Val Cys Leu Ala Val Tyr Phe Val Leu Thr Leu Lys Asp
        195                 200                 205
Gly Ala Gly Tyr Val Phe Ser Val Phe Phe Ala Val Tyr Ala Phe Ala
    210                 215                 220
Ile Ala Gly Tyr Glu His Ile Ile Ala Asn Ile Tyr Thr Leu Asn Ile
225                 230                 235                 240
Ala Leu Met Val Asn Thr Lys Ile Thr Val Tyr Gln Ala Tyr Ile Lys
                245                 250                 255
Asn Leu Leu Pro Thr Leu Leu Gly Asn Tyr Ile Ala Gly Ala Ile Val
            260                 265                 270
Leu Gly Leu Pro Leu Tyr Phe Ile Tyr Lys Glu His Tyr Tyr Asn Phe
        275                 280                 285
Glu Arg Ser Lys Arg Asp Asn Asn Asp Ala Gln Met Lys Ser Leu Ser
    290                 295                 300
Ile Glu Leu Arg Asn
305

<210> 2
<211> 412
<212> PRT
<213> Toxoplasma gondii

<220>
<223> TgFNT1_TGGT1_209800_ToxoDB

<400> 2
Met Val Val Thr Ala Ser Pro Asp Thr Tyr Leu His Val Ile Asp Tyr
1               5                   10                  15
Gly Leu Lys Lys Val Arg Leu Arg Phe Asp Arg Leu Leu Leu Gln Ala
            20                  25                  30
Phe Met Ala Gly Val Tyr Ile Gly Met Ala Gly Asn Ala Cys Ile Ser
        35                  40                  45
Leu Ala Gly Gly Phe Ser Thr Asp Pro Ala Asp Pro Lys Ala Ile Thr
    50                  55                  60
Ala Gly Val Gln Lys Phe Ile Tyr Ala Ser Ile Phe Pro Val Ala Phe
65                  70                  75                  80
Ile Ala Ile Ile Met Thr Gly Ala Glu Leu Phe Thr Gly Asn Thr Met
                85                  90                  95
Thr Met Leu Ile Cys Trp Phe Glu Arg Arg Ile Thr Ile Trp Gln Leu
            100                 105                 110
Leu Gln Asn Trp Ala Gly Ser Phe Leu Gly Asn Trp Leu Gly Thr Met
        115                 120                 125
Phe Ser Ala Tyr Phe Leu Thr Tyr Leu Cys Cys Pro Phe Asp His Asp
    130                 135                 140
Pro Tyr Leu Ser Tyr Leu Asn Tyr Thr Ala Ala Ser Lys Val Ser Tyr
145                 150                 155                 160
Gly Trp Gly Ser Cys Phe Leu Arg Gly Val Gly Cys Asn Thr Trp Val
                165                 170                 175
Cys Leu Ala Val Trp Phe Val Val Ala Cys Asp Asp Ala Ala Gly Lys
            180                 185                 190
Ile Leu Ala Leu Trp Phe Pro Ile Val Ala Phe Val Leu Ser Ser Tyr
        195                 200                 205
Glu His Ile Ile Ala Asn Leu Tyr Thr Leu Gln Leu Cys Ala Met Leu
    210                 215                 220
Gly Val Asp Thr Ser Leu Ala Asp Met Ile Ala Phe Asn Leu Leu Pro
225                 230                 235                 240
Thr Leu Leu Gly Asn Leu Phe Gly Gly Cys Gly Leu Ile Gly Met Val
                245                 250                 255
Tyr Phe Tyr Asn Phe Tyr Pro Val Val Gly His Gly Asp Asp Ala Ala
            260                 265                 270
Glu Gly Ser Ile Cys Gly Ser Ser Glu Lys Glu Glu Cys Pro Ser Leu
        275                 280                 285
Val Gly Val Pro Arg Gly Ala Ser Val Asn Ser Leu Ala Val Ser Ala
    290                 295                 300
Ile Pro Ser Val Phe Ser Ala Pro Arg Gly Gln Arg Glu Ser Phe Ala
305                 310                 315                 320
Gly Glu Ser Ser Thr Leu Val Met Gly Asp Ile Lys Arg Gln Arg Ser
                325                 330                 335
Met Ala Ser Thr Arg Lys Leu Gly Gly Gly Ala Asp Lys Lys Asp Val
            340                 345                 350
Gln Leu Thr Val Arg Gln Phe Asp Glu Thr Glu Met Gln Ser Thr Met
        355                 360                 365
Glu Asp Met Phe Gly Leu Glu Asp Pro Arg Asn Ala Pro Lys Gly Asn
    370                 375                 380
Pro Gly Thr Asn Pro Pro Ser Lys Ser Pro Glu Ser Ser Ala Thr Gly
385                 390                 395                 400
Gly Thr Gly Ala Ala Ala Ser Pro Thr Ala Thr Ser
                405                 410

<210> 3
<211> 463
<212> PRT
<213> Toxoplasma gondii

<220>
<223> TgFNT3_TGGT1_292110_ToxoDB

<400> 3
Met Cys Ser Ile Pro Pro Leu Arg Leu Leu Glu Val Val Asp Asp Leu
1               5                   10                  15
Thr Cys Leu Val Asn Ser Ser Gln Tyr Ser Arg Trp Asp Phe Phe Thr
            20                  25                  30
Phe Thr Leu Pro Gly Ser Thr Leu Pro Met Arg Lys Glu Phe Thr Ser
        35                  40                  45
Ser Cys Lys Gly Ser Phe Ser Asn His Pro Val Pro Asp His Pro Cys
    50                  55                  60
Lys Leu Val Val Phe Val Arg Pro Lys Met Val Val Thr Ala Gly Ala
65                  70                  75                  80
Asp Ala Tyr Leu Lys Ile Leu Glu Tyr Gly Val Lys Lys Thr Gln Leu
                85                  90                  95
Arg Ile Asp Arg Leu Leu Leu Gln Ala Phe Met Ala Gly Ile Phe Val
            100                 105                 110
Ala Met Ala Gly His Cys Cys Thr Val Leu Ala Gly Ser Tyr Pro Thr
        115                 120                 125
Asp Pro Gly Asp Pro Leu Ala Val Ala Lys Pro Thr Gln Lys Phe Ile
    130                 135                 140
Tyr Gly Ala Leu Phe Pro Val Ala Phe Ile Cys Ile Ile Leu Thr Gly
145                 150                 155                 160
Ala Glu Leu Phe Thr Gly Asn Thr Met Thr Met Leu Ile Cys Tyr Phe
                165                 170                 175
Gln Lys Arg Val Thr Met Leu Gln Leu Gly Val Asn Trp Leu Gly Ser
            180                 185                 190
Leu Ala Gly Asn Trp Leu Gly Ala Leu Phe Gly Ala Tyr Phe Leu Ser
        195                 200                 205
Tyr Leu Thr Gly Ala Leu Gly Asp Glu His Val Arg Gln Phe Leu Phe
    210                 215                 220
Arg Thr Cys Val Asn Lys Ile Ser Tyr Gly Trp Gly Glu Cys Phe Leu
225                 230                 235                 240
Arg Gly Val Gly Cys Asn Thr Phe Val Cys Leu Ala Val Trp Ala Val
                245                 250                 255
Ile Ala Ser Glu Asn Val Ala Gly Lys Val Leu Val Met Trp Phe Pro
            260                 265                 270
Ile Val Ala Phe Cys Val Gly Gly Tyr Glu His Ile Ile Ala Asn Met
        275                 280                 285
Tyr Thr Leu Gln Ala Gly Leu Met Ala Gly Ala Pro Val Ala Ile Leu
    290                 295                 300
Asp Val Ile Ala Phe Asn Phe Leu Pro Thr Leu Leu Gly Asn Ile Val
305                 310                 315                 320
Gly Gly Cys Leu Leu Val Gly Ala Val Tyr Ala Tyr Asn Phe Tyr Pro
                325                 330                 335
Thr Leu Ser Tyr Thr Glu Thr Thr Gly Ala Lys Val Tyr Val Gln Glu
            340                 345                 350
Val Gly Pro Val Leu Asp Arg Arg Ser Ser Met Gln Val Ser Met Thr
        355                 360                 365
Glu Arg Glu Pro Asp Gly Gln Val Val Thr Glu Tyr Glu Ala Val Pro
    370                 375                 380
Phe Glu Ser Phe Gly Gly Glu Tyr Ile Val Asn Lys His Ala Thr Met
385                 390                 395                 400
Ala Ala Pro Ile Pro Ser Arg Ala Ser Ser Phe Leu Tyr Pro Phe Gln
                405                 410                 415
Trp Gln Arg Gln Arg Ser Gln Ser Gly Asn Leu Ser Thr His Ala Arg

420                 425                 430
Leu Asp Leu Pro Asn Arg Pro Val Glu Pro Pro Ser Asp Gly Leu Glu
        435                 440                 445
Val Thr Pro Gln Ser Gln Thr Ala Glu Ser Val Ala Gln Gln Val
    450                 455                 460

<210> 4
<211> 501
<212> PRT
<213> Toxoplasma gondii

<220>
<223> TgFNT2_TGGT1_229170_ToxoDB

<400> 4
   Met Val Leu Ala Ala Ser Pro Glu Ala Tyr Arg Lys Val Ile Glu Tyr
   1               5                   10                  15
   Gly Ile Lys Lys Thr Lys Leu Arg Ile Asp Arg Leu Phe Leu Gln Ala
               20                  25                  30
   Ile Met Ala Gly Ile Tyr Val Gly Met Ala Gly His Ala Cys Thr Ala
           35                  40                  45
   Leu Ala Gly Ala Tyr Ser Thr Asp Pro Ala Asn Pro Leu Ala Val Ser
       50                  55                  60
   Lys Ala Thr Gln Lys Phe Leu Tyr Ala Ser Leu Phe Pro Val Ala Phe
   65                  70                  75                  80
   Ile Ala Ile Ile Phe Thr Gly Ala Glu Leu Phe Thr Gly Asn Thr Met
                   85                  90                  95
   Thr Met Leu Val Cys Leu Leu Glu Arg Arg Val Thr Ala Leu Gln Leu
               100                 105                 110
   Cys Ile Asn Trp Ile Cys Ser Leu Val Gly Asn Trp Ala Gly Ala Leu
           115                 120                 125
   Phe Ala Ala Tyr Phe Leu Ser Tyr Leu Pro Gly Val Leu Gln Asp Pro
       130                 135                 140
   Asp His Leu His Tyr Leu Glu Asp Val Ala Ala His Lys Thr Glu Leu
   145                 150                 155                 160
   Ser Phe Leu Gln Cys Phe Cys Leu Ala Val Gly Cys Asn Thr Phe Val
                   165                 170                 175
   Cys Leu Ala Val Trp Phe Val Ile Ala Ser Asp Asp Ala Ala Gly Lys
               180                 185                 190
   Ile Met Ser Met Trp Phe Pro Ile Val Ser Phe Cys Val Ala Gly Tyr
           195                 200                 205
   Glu His Ile Ile Ala Asn Phe Tyr Thr Leu Gln Cys Ala Leu Met His
       210                 215                 220
   Gly Val Gly Pro Gly Val Gly Thr Val Ile Leu Lys Asn Phe Ile Pro
   225                 230                 235                 240
   Thr Leu Leu Gly Asn Ile Val Gly Gly Cys Gly Leu Val Gly Ala Val
                   245                 250                 255
   Tyr Trp Tyr Asn Phe Tyr Pro Thr Val Cys Val Val Gln Glu Ala Arg
               260                 265                 270
   Gln Pro Leu Pro Leu Ser Glu Asn Ala Pro Ser Ser Thr Arg Gln Val
           275                 280                 285
   Val Ala Asp Leu Phe Ser Leu Trp Gly Arg Glu Ser Ser Thr Pro Gly
       290                 295                 300
   Val Ser Ala Ser Pro Pro Asp Ala Ala Thr Asn Ala Gly Cys Ser Ala
   305                 310                 315                 320
   Leu Asp Pro Pro Arg Asn Ala Leu Leu Ala Ala Gly Lys Asn Phe Gly
                   325                 330                 335
   Asn Leu Ser Ala Gly Asp Arg Gly Ala Leu Ala Glu Gly Ile Pro Gly
               340                 345                 350
   Gly Ala Cys Glu Asp Cys Leu Leu Val Pro Arg Ala Ser Phe Gly Gly
           355                 360                 365
   Glu Tyr His Pro Pro Gln Gln Gly Asp Ala Gly Arg Trp Cys Lys Pro
   370                 375                 380
   Ser Lys Ala Ala Val Gly Ser Gly Gly Val Leu Cys His Val Gln Ser
   385                 390                 395                 400
   Pro Ala Ala Leu Glu Ala Val Ser Asn Ser Pro Leu Arg Glu Asn Ser
                   405                 410                 415
   Gly Val Pro Ser Gly Gly Leu Leu Leu Cys Glu Gly Arg Val Arg Arg
               420                 425                 430
   Ser Ser Arg Glu Arg Glu Pro Glu Arg Gly Gly Glu Glu Glu Glu Gly
           435                 440                 445
   Ala Ser Pro Glu Glu Glu His Pro Ala Val Thr Leu Ser Ile Pro Pro
       450                 455                 460
   Thr Asp Phe His Pro His Val Pro Arg Glu Val Glu Gln Ser Ser Leu
   465                 470                 475                 480
   Leu Glu Glu Thr Arg Val Ala Ala Glu Asn Ser Ala Leu Glu Glu His
                   485                 490                 495
   Pro Ala Ser Thr Ile
               500

<210> 5
<211> 356
<212> PRT
<213> Entamoeba histolytica

<220>
<223> EhFNT_EHI_198990_AmoebaDB

<400> 5
Met Pro Arg Glu Lys Pro Arg Ala Asp Glu Ile Ala Ile Glu Met Met
1               5                   10                  15
Ser Val Cys Glu Asp Glu Thr Glu Val Glu Gln Asp Pro Arg Glu Leu
            20                  25                  30
Tyr Glu Glu Glu Met Lys Glu Gln Gln Gln Ile Asp Cys Ser Lys Gln
        35                  40                  45
Gln Lys Glu Val Val Ala Ile Glu Glu Leu Glu Lys Arg Asn Ile Asn
    50                  55                  60
Lys His Phe Phe Ser Ile Gln Pro Asn Thr Gln Ile Pro Val Ile Ser
65                  70                  75                  80
Ser Asn Tyr Ile Ala Pro Val Asp Thr Ser Arg Leu Leu Val Leu Ile
                85                  90                  95
Gly Lys Thr Lys Ala Thr Tyr Pro Ile Met Lys Met Phe Ser Leu Ser
            100                 105                 110
Val Leu Ala Gly Met Leu Leu Ser Val Gly Gly Leu Leu Ser Ile Thr
        115                 120                 125
Ile Gly Lys Gly Ile Pro Ser Ser Asp Ile Gly Ile Gln Lys Ile Val
    130                 135                 140
Phe Gly Phe Phe Asn Ser Val Gly Leu Asn Leu Val Val Leu Cys Gly
145                 150                 155                 160
Gly Glu Leu Phe Thr Ser Asn Cys Ala Phe Leu Ile Pro Gly Phe Met
                165                 170                 175
Glu Gly Ala Tyr Ser Arg Trp Leu Phe Phe Lys Thr His Phe Val Val
            180                 185                 190
Tyr Phe Gly Asn Leu Val Gly Ser Ile Phe Val Ser Thr Tyr Phe Gly
        195                 200                 205
Lys Leu Leu Gly Ser Phe Glu Ser Pro Met Tyr Leu Ser Ala Val Lys
    210                 215                 220
Gln Ile Gly Glu Thr Lys Val Ala Met Asn Trp Gly Arg Ala Leu Leu
225                 230                 235                 240
Ser Gly Ile Gly Cys Asn Trp Leu Val Cys Cys Ala Val Tyr Phe Ser
                245                 250                 255
Ala Ser Ala Lys Asp Leu Leu Ser Lys Leu Val Val Ile Ser Phe Leu
            260                 265                 270
Val Leu Thr Phe Ala Ser Leu Glu Phe Glu Asn Cys Val Gly Asn Met

275                 280                 285
Phe Leu Leu Ser Leu Ser His Met Tyr Gly Gly Asn Phe Thr Leu Gly
    290                 295                 300
Gln Trp Ile Leu Asn Asn Leu Ile Pro Val Ser Ile Gly Asn Phe Ile
305                 310                 315                 320
Gly Gly Thr Phe Leu Leu Gly Ile Pro Leu Trp Tyr Val His Val Ser
                325                 330                 335
Asn Val Tyr Asn Ile Pro Phe Leu Asp Pro Leu Tyr Gln Gln Ser Gln
            340                 345                 350
Ala Lys Thr Gln
        355

<210> 6
<211> 308
<212> PRT
<213> Naegleria fowleri

<220>
<223> NfFNT1, VFQX01000074.1, GenBank

<400> 6
Met Ala Leu Ser Gly Glu Lys Lys Ser Lys Glu Ser Leu Asp Gln Thr
1               5                   10                  15
Ile Ile Leu Ala Leu Leu Ser Gly Val Phe Val Ala Phe Gly Ala Ile
            20                  25                  30
Ala Ser Phe Lys Thr Gly Gly Asn Met Pro Gly Trp Asp Val Asp Asn
        35                  40                  45
Pro Gly Tyr Gly Lys Leu Ile Phe Ser Ala Val Phe Thr Val Gly Leu
    50                  55                  60
Met Leu Val Ile Val Ser Gly Ser Glu Leu Phe Thr Gly Asn Ala Met
65                  70                  75                  80
Val Met Ile Val Ala Met Leu Arg Lys Lys Val Thr Ala Trp His Val
                85                  90                  95
Val Lys Asn Leu Cys Leu Ser Leu Ala Phe Asn Val Leu Gly Ala Phe
            100                 105                 110
Met Thr Ile Tyr Phe Phe Met Tyr Leu Pro Thr Thr Glu Lys Glu Leu
        115                 120                 125
His Thr Pro Ala Gly Phe Val Thr Tyr Ala Lys Lys Ile Gly Glu Ser
    130                 135                 140
Lys Val Ser Lys Gly Ala Gly Val Thr Ile Leu Leu Gly Ile Gly Cys
145                 150                 155                 160
Asn Trp Leu Val Cys Leu Ala Val Tyr Met Thr Tyr Ala Ser Asn Val
                165                 170                 175
Leu Val Asp Lys Leu Val Gly Leu Phe Met Pro Ile Leu Ala Phe Val
            180                 185                 190
Ala Gly Gly Tyr Glu His Cys Val Ala Asn Gly Ala Phe Ile Pro Leu
        195                 200                 205
Ala Met Leu Tyr Gly Ser Asp Ile Thr Met Tyr Asp Phe Phe Val Val
    210                 215                 220
Asn Leu Met Pro Ala Thr Leu Gly Asn Leu Leu Gly Gly Ala Ile Phe
225                 230                 235                 240
Val Gly Met Thr Phe Trp Tyr Thr His Gly Val Arg Met Glu His Leu
                245                 250                 255
Tyr Phe Phe Asp Glu His Phe Thr Leu Arg Met Arg Ala Ala Ile Ala
            260                 265                 270
Gln Pro Ile Leu Asn Val Trp Asp Arg Leu Gln Lys Phe Asn Pro Phe
        275                 280                 285
Leu Lys Lys Glu Glu Ala Ile Ile Ser Ala Gln Asn Ile Glu Leu Pro
    290                 295                 300
Asp His Arg Val
305

<210> 7
<211> 310
<212> PRT
<213> Naegleria fowleri

<220>
<223> NfFNT2, Sequence_ID: VFQX01000001.1, GenBank

<400> 7
   Met Lys Thr Glu Gly Val Arg Lys Ser Lys Leu Thr Val Asp Gln Met
   1               5                   10                  15
   Ile Val Leu Ser Leu Phe Ala Gly Ile Phe Lys Gly Val Ser Cys Thr
               20                  25                  30
   Leu Ser Leu Phe Thr Ala Gly Asn Ser Arg Lys Leu Glu Glu Glu Phe
           35                  40                  45
   Pro Gly Leu Gln Lys Phe Phe Phe Gly Ala Val Phe Pro Ile Gly Leu
       50                  55                  60
   Ile Leu Ile Ser Ala Thr Gly Ala Glu Leu Phe Thr Ala Asn Val Leu
   65                  70                  75                  80
   Val Val Leu Val Gly Leu Phe Ser Arg Lys Trp Arg Ala Lys Lys Phe
                   85                  90                  95
   Ile Ile Gln Phe Ala Gln Phe Leu Lys Asn Leu Val Leu Ser Tyr Val
               100                 105                 110
   Phe Asn Met Leu Gly Ala Phe Val Met Ala Tyr Phe Phe Val Tyr Leu
           115                 120                 125
   Ile Gln Pro Cys Cys Asn Ala His Trp Ile Glu Tyr Val Lys His Leu
       130                 135                 140
   Ala Glu Thr Lys Ser Ser Lys Ser Phe Gly Ala Cys Leu Ala Ser Gly
   145                 150                 155                 160
   Ile Ile Cys Asn Met Leu Val Thr Met Ala Thr Tyr Asn Ser His Ala
                   165                 170                 175
   Ala Thr Thr Leu Glu Gly Lys Ile Leu Gly Ile Trp Phe Pro Ile Met
               180                 185                 190
   Thr Phe Val Ser Ile Gly Leu Glu His Cys Ile Ala Asn Ala Tyr Phe
           195                 200                 205
   Ile Pro Leu Gly Met Leu Tyr Gly Ala Asn Val Ser His Tyr Asp Phe
       210                 215                 220
   Phe Ile Gly Asn Leu Leu Pro Ala Thr Ile Gly Asn Ile Ile Gly Gly
   225                 230                 235                 240
   Ala Phe Val Ile Gly Cys Leu Leu Tyr Phe Val Tyr Asp Tyr Arg Asn
                   245                 250                 255
   Arg His Asn Arg Val Leu Phe Asn Phe Leu Glu Leu Thr Trp Ser Lys
               260                 265                 270
   Ile Lys Glu Lys Tyr Lys Gln Ser Arg Asn Ser Asn Gln Pro Glu Arg
           275                 280                 285
   Val Ser Thr Asn Glu Arg Lys Glu Ser Thr Asp Ser Val Leu Ser Asp
       290                 295                 300
   Leu Glu Met Gly Tyr Arg
   305                 310

<210> 8
<211> 228
<212> PRT
<213> Naegleria fowleri

<220>
<223> partial sequence of NfFNT3, Sequence_ID: VFQX01000009.1, GenBank

<400> 8
Met Ile Gly Ser Phe Met Val Ala Tyr Phe Phe Leu Tyr Leu Thr Gln
1               5                   10                  15

Pro Cys Cys Asp Ala Pro Trp Ile Ala Tyr Ala Lys Lys Ile Ala Leu
            20                  25                  30
Ser Lys Val Ser Lys Thr Phe Gly Gln Asn Val Leu Leu Gly Ile Val
        35                  40                  45
Cys Asn Met Met Leu Thr Leu Ser Ile Tyr Ile Ser Phe Ala Ala Thr
    50                  55                  60
Thr Val Glu Gly Lys Ile Ile Gly Val Trp Phe Pro Val Met Gly Phe
65                  70                  75                  80
Ala Ser Ser Gly Phe Glu His Cys Ile Ala Asn Ala Phe Phe Ile Pro
                85                  90                  95
Leu Gly Met Leu Phe Gly Glu Asp Ile Ser Leu Gln Asp Phe Leu Val
            100                 105                 110
Gln Asn Leu Leu Pro Thr Thr Ile Gly Asn Leu Ile Gly Gly Ser Phe
        115                 120                 125
Ile Val Gly Leu Leu Leu Tyr Tyr Val His Asp Leu Arg His Arg His
    130                 135                 140
Arg Arg Phe Leu Phe Asn Thr Ile Glu Arg Leu Trp Ser Tyr Leu Lys
145                 150                 155                 160
Gln Arg Tyr Ile His Gln Arg Leu Pro His Ser Ser Ser Thr Thr Pro
                165                 170                 175
Leu Ser Ser Thr Thr Ala Ala Thr Ser His Leu Gly Thr Gly Glu Ser
            180                 185                 190
Ser Ser Lys Arg Thr Asp Ser Pro Ser Thr Gln Gln Pro Ser Asn Lys
        195                 200                 205
Ser Ser Leu Glu Phe Thr Glu Glu Val Glu Met Val Asp Ile Asn Val
    210                 215                 220
Glu Asn Lys Val
225

<210> 9
<211> 313
<212> PRT
<213> Plasmodium vivax

<220>
<223> FNT

<400> 9
Met Thr Lys Gly Ser Lys Tyr Thr Ile Asp Pro Ile Ser Val Lys Thr
1               5                   10                  15
Ala Cys Thr Ser Glu Glu Ser Tyr Ile Arg Cys Val Glu Tyr Gly Lys
            20                  25                  30
Gly Lys Ala His Tyr Pro Asn Leu Ser Leu Leu Ala Lys Ala Ile Leu
        35                  40                  45
Ala Gly Val Phe Val Gly Val Cys Ala His Ala Ser Gly Ile Ala Gly
    50                  55                  60
Gly His Phe Tyr Tyr His Lys Leu Arg Glu His Val Gly Ile Ser Met
65                  70                  75                  80
Ser Ala Phe Val Tyr Gly Phe Thr Phe Pro Ile Ala Phe Leu Cys Ile
                85                  90                  95
Ile Ala Thr Gly Ser Asp Leu Phe Thr Gly Asn Thr Leu Ala Val Thr
            100                 105                 110
Thr Ala Leu Leu Gln Arg Lys Val Thr Leu Leu Glu Tyr Leu Arg Val
        115                 120                 125
Met Ser Ile Ser Leu Phe Gly Asn Tyr Val Gly Ala Val Ser Phe Ala
    130                 135                 140
Phe Phe Val Ser His Leu Ser Gly Ala Phe Lys Lys His Glu Glu Ile
145                 150                 155                 160
Gly Lys Asn His Ile Phe Gln Phe Leu Asn Asp Ile Ala Glu Lys Lys
                165                 170                 175
Val Ser His Thr Phe Val Gln Cys Val Cys Leu Ala Ile Gly Cys Asn
            180                 185                 190

Ile Phe Val Cys Leu Ala Val Tyr Phe Val Leu Thr Ile Lys Asp Gly
        195                 200                 205
Ser Gly Met Val Phe Ser Val Phe Phe Ala Val Tyr Ala Phe Ala Ile
    210                 215                 220
Ala Gly Tyr Glu His Ile Ile Ala Asn Met Tyr Thr Leu Asn Leu Ala
225                 230                 235                 240
Leu Met Ile Glu Ala Asn Val Asp Trp Thr Lys Ala Tyr Val Asp Asn
                245                 250                 255
Leu Leu Pro Thr Leu Ile Gly Asn Tyr Ile Ala Gly Ala Ile Val Leu
            260                 265                 270
Ala Cys Pro Leu Phe Tyr Ile Tyr Arg His Ser Tyr Ser Asp Tyr Glu
        275                 280                 285
Lys Thr Arg Gly Asp Gly Gly Asn Ser Gly Leu Lys Ser Leu Ser Ile
    290                 295                 300
Glu Met Gln Asn Gly Ser Ser Gly Arg
305                 310

<210> 10
<211> 314
<212> PRT
<213> Plasmodium knowlesi

<220>
<223> FNT

<400> 10
Met Ser Lys Gly Lys Ser Lys Tyr Val Ile Asp Pro Ile Ser Val Lys
1               5                   10                  15
Thr Ala Cys Thr Ser Glu Glu Ser Tyr Ile Arg Cys Val Glu Tyr Gly
            20                  25                  30
Lys Gly Lys Ala His Tyr Pro Asn Leu Ser Leu Leu Ala Lys Ala Ile
        35                  40                  45
Leu Ala Gly Val Phe Val Gly Val Cys Ala His Ala Ser Gly Ile Ala
    50                  55                  60
Gly Gly His Phe Tyr Tyr His Lys Leu Arg Glu Tyr Val Gly Ile Ser
65                  70                  75                  80
Met Ser Ala Phe Val Tyr Gly Phe Thr Phe Pro Ile Ala Phe Leu Cys
                85                  90                  95
Ile Ile Ala Thr Gly Ser Asp Leu Phe Thr Gly Asn Thr Leu Ala Val
            100                 105                 110
Thr Thr Ala Leu Leu Gln Arg Lys Val Ser Leu Leu Gln Tyr Leu Arg
        115                 120                 125
Val Met Ser Ile Ser Leu Phe Gly Asn Tyr Leu Gly Ala Val Ser Phe
    130                 135                 140
Ala Phe Phe Val Ser His Leu Ser Gly Ala Tyr Glu Lys His Thr Asp
145                 150                 155                 160
Val Thr Lys Asn His Ile Phe Gln Phe Leu Asn Asp Ile Ala Glu Lys
                165                 170                 175
Lys Ile Ser His Thr Phe Ile Gln Cys Ile Cys Leu Ala Ile Gly Cys
            180                 185                 190
Asn Ile Phe Val Cys Leu Ala Val Tyr Phe Val Leu Thr Ile Lys Asp
        195                 200                 205
Gly Ser Gly Met Val Phe Ser Val Phe Phe Ala Val Tyr Ala Phe Ala
    210                 215                 220
Ile Ala Gly Tyr Glu His Ile Ile Ala Asn Met Tyr Thr Leu Asn Leu
225                 230                 235                 240
Ala Leu Met Val Glu Ala Lys Val Thr Trp Ser Lys Val Tyr Phe His
                245                 250                 255
Asn Leu Leu Pro Thr Leu Ile Gly Asn Tyr Ile Ala Gly Ala Leu Val
            260                 265                 270
Leu Ala Cys Pro Leu Phe Tyr Ile Tyr Arg Asn Ser Tyr Arg Asp Tyr
        275                 280                 285

Glu Arg Thr Arg Gly Asp Gly Ser Asn Cys Gly Leu Arg Ser Leu Ser
    290                 295                 300
Ile Glu Met Gln Asn Gly Ser Asn Gly Asn
305                 310

<210> 11
<211> 313
<212> PRT
<213> Plasmodium ovale

<220>
<223> FNT

<400> 11
Met Pro Lys Ser Asn Thr Lys Tyr Val Ile Asp Pro Leu Ser Val Lys
1               5                   10                  15
Thr Ser Cys Ser Ser Glu Glu Ser Tyr Ile Arg Cys Val Glu Tyr Gly
            20                  25                  30
Lys Ser Lys Ala His Tyr Ser Ser Leu Ile Leu Leu Ala Lys Ala Ile
        35                  40                  45
Leu Ala Gly Val Phe Val Gly Val Cys Ala His Ala Ser Gly Ile Ala
    50                  55                  60
Gly Gly Leu Phe Tyr Tyr His Lys Leu Arg Glu Tyr Val Gly Ala Ser
65                  70                  75                  80
Met Ser Ala Phe Val Tyr Gly Phe Thr Phe Pro Ile Ala Phe Leu Cys
                85                  90                  95
Ile Ile Cys Thr Gly Ser Asp Leu Phe Thr Gly Asn Thr Leu Ala Val
            100                 105                 110
Thr Thr Ala Leu Leu His Gly Lys Val Ser Cys Leu Glu Tyr Val Arg
        115                 120                 125
Val Met Cys Ile Ser Leu Phe Gly Asn Tyr Val Gly Ala Val Ser Phe
    130                 135                 140
Ala Phe Phe Val Ser Tyr Gly Ser Gly Ala Phe His Lys Lys Glu Gln
145                 150                 155                 160
Val Asp Lys Asn His Ile Phe Gln Phe Leu Asn Asp Ile Ala Val Lys
                165                 170                 175
Lys Val Asn His Thr Phe Val Glu Cys Ile Cys Leu Ala Ile Gly Cys
            180                 185                 190
Asn Ile Phe Val Cys Leu Ala Val Tyr Phe Val Leu Ser Ile Lys Asp
        195                 200                 205
Gly Ser Gly Met Val Phe Ser Val Phe Phe Ala Val Tyr Ala Phe Ala
    210                 215                 220
Ile Ala Gly Tyr Glu His Ile Ile Ala Asn Ile Tyr Thr Leu Asn Ile
225                 230                 235                 240
Ser Leu Met Ile Asp Thr Glu Val Ser Phe Thr Gln Val Tyr Phe Lys
                245                 250                 255
Asn Leu Leu Pro Thr Leu Ile Gly Asn Tyr Ile Ala Gly Ala Leu Val
            260                 265                 270
Leu Ala Cys Pro Leu Phe Phe Ile Tyr Arg Ser Tyr Tyr Ile Asn Tyr
        275                 280                 285
Glu Lys Met Asn Glu Pro Ser Gly Gly Ser Leu Arg Ser Ile Ser Ile
    290                 295                 300
Glu Met Lys Asn Asp Gly Gly Ala Thr
305                 310

<210> 12
<211> 314
<212> PRT
<213> Plasmodium malariae

<220>

<223> FNT

<400> 12
Met Gln Lys Ser Thr Ser Lys Tyr Val Ile Asp Pro Ile Ser Ile Lys
1               5                   10                  15
Thr Asn Cys Ser Ser Glu Glu Ser Tyr Ile Arg Cys Val Glu Tyr Gly
            20                  25                  30
Lys Gly Lys Ala His Tyr Arg Asn Leu Ile Leu Leu Ala Lys Ala Ile
        35                  40                  45
Leu Ala Gly Val Phe Val Gly Val Cys Ala His Ala Ser Gly Ile Ala
    50                  55                  60
Gly Gly Leu Phe Tyr Tyr His Lys Leu Arg Glu Tyr Val Gly Ile Ser
65                  70                  75                  80
Met Ser Ala Phe Val Tyr Gly Phe Thr Phe Pro Ile Ala Phe Leu Cys
                85                  90                  95
Ile Ile Cys Thr Gly Ser Asp Leu Phe Thr Gly Asn Thr Leu Ala Val
            100                 105                 110
Thr Thr Ala Leu Leu Gln Lys Lys Leu Gly Leu Leu Cys Tyr Met Arg
        115                 120                 125
Val Met Cys Ile Ser Leu Val Gly Asn Tyr Ile Gly Ala Val Ala Phe
    130                 135                 140
Ala Phe Phe Val Ser Tyr Gly Ser Gly Ala Phe Ser Ile Asn Thr Asp
145                 150                 155                 160
Thr Ser Lys Asn His Ile Phe Gln Phe Leu Asn Asp Ile Ala Ile Lys
                165                 170                 175
Lys Val Ser His Ser Phe Ile Glu Cys Ile Cys Leu Ala Ile Gly Cys
            180                 185                 190
Asn Ile Phe Val Cys Leu Ala Val Tyr Phe Val Leu Ser Ile Lys Asp
        195                 200                 205
Gly Ser Gly Leu Val Phe Ser Val Phe Phe Ala Val Tyr Ala Phe Ala
    210                 215                 220
Ile Ala Gly Tyr Glu His Ile Ile Ala Asn Ile Tyr Thr Leu Asn Leu
225                 230                 235                 240
Ala Leu Met Ile Ser Asn Asp Ile Ser Phe Thr Gln Val Tyr Phe Lys
                245                 250                 255
Asn Leu Leu Pro Thr Leu Ile Gly Asn Tyr Ile Ala Gly Gly Leu Val
            260                 265                 270
Leu Ala Phe Pro Leu Phe Phe Ile Tyr Arg Ser Cys Tyr Tyr Asp Tyr
        275                 280                 285
Asp Lys Met Asn Asp Glu Leu Asn Thr Val Val Leu Lys Thr Leu Ser
    290                 295                 300
Leu Glu Leu Gln Asn Glu Ser Asn His Ile
305                 310

## Description

The present invention relates to active substances that are active against protozoa, especially against Plasmodium spp., e.g. against Plasmodium falciparum, Plasmodium vivax, P. malariae, P. ovale , P. knowlesi, against Toxoplasma spp., e.g. Toxoplasma gondii, against Entamoeba histolytica, against Naegleria fowleri, and relates to a method of treatment and/or prophylaxis of an infection by a protozoon. The active substances are suitable for use in the treatment of and/or for use in prophylaxis of an infection by a protozoon.

The active substances have the advantage of having activity against wild-type protozoa, especially against Plasmodium spp. and against Toxoplasma spp. and against Entamoeba histolytica and against Naegleria fowleri, and of having activity also against a mutant of P. falciparum that has been identified as resistant against other anti-malarial active substances.

Further, the active substances for use against infections by protozoa, which active substances are compounds that are suitable for anti-malarial therapy and/or prophylaxis and are suitable for anti-toxoplasmosis therapy and/or prophylaxis, and are suitable for therapy and/or prophylaxis against Entamoeba histolytica infection, and are suitable for therapy and/or prophylaxis against Naegleria fowleri infection, and are suitable for therapy and/or prophylaxis against infection by the cattle parasite Babesia bovis, have the advantage of being available by chemical synthesis, which can be carried out by non-complicated synthetic routes.

Accordingly, the invention also relates to a production method for producing the active substances of the invention.

The active substances of the invention are dispersible and preferably water-soluble, and therefore the active substances can be contained in an aqueous pharmaceutical formulation or in a dry, water-soluble or water-miscible pharmaceutical formulation.

### State of the art

WO2017/211988 A1 describes active substances for treatment of infections by protozoa, e.g. 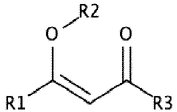 wherein R1 can be a perfluoro-C₁- to C₄-alkyl, R2 can be H or a C₁- to C12-alkyl, and R3 can be an aromatic ring, exemplified by a phenyl ring. The active substances are described as inhibitors of the formiate-nitrite-transporter protein (FNT) of protozoa.

Golldack et al., PLOS Pathogens, DOI: 10.1371/journal.ppat.1006172 (2017) describes 5,5,6,6,6-pentafluoro-4-hydroxyhex-3-en-2-one as a pharmacophore for use against malaria.

### Object of the invention

It is an object of the invention to provide improved active substances for use in the treatment or prophylaxis of infections by protozoa, especially against malaria. A preferred object is the provision of active substances that are active against protozoa, especially malaria, that have developed a resistance, and to more preferably provide active substances for use in the treatment of infections by protozoa, against which active substances protozoa, e.g. Plasmodium spp., can be expected to not develop resistance.

### Description of the invention

The present invention achieves the object by the features of the claims, and specifically by providing compounds as active substances for treatment and/or prophylaxis of infections by protozoa, the active substances comprising or consisting of compound I 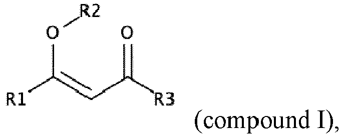
wherein R1 is perfluoro-Ci- to C₄-alkyl, straight chain or branched, especially trifluoromethyl, pentafluoroethyl, heptafluoropropyl, nonafluorobutyl, preferably pentafluoroethyl, or solvates or salts thereof,
wherein R2 is H or a C₁- to C₁₂-alkyl, e.g. methyl, ethyl, *n*-propyl, *n*-butyl, *n*-pentyl, *n*-hexyl, *n*-octyl, n-decyl, *n*-dodecyl, *iso*-propyl, *iso*-butyl, *tert*-butyl, 2,2-dimethylpropyl or cyclohexyl, preferably H or ethyl, or R2 is a carbonyl group with H or a C₁- to C₁₂-alkyl, which is e.g. methyl, ethyl, *n*-propyl, *n*-butyl, *n*-pentyl, *n*-hexyl, *n*-octyl, *n*-decyl, *n*-dodecyl, *iso*-propyl, *iso*-butyl, *tert*-butyl, 2,2-dimethylpropyl or cyclohexyl, or R2 is a carbonyl group with H or a C₁- to C₁₂-alkyl, which is e.g. methyl, ethyl, *n*-propyl, *n*-butyl, *n*-pentyl, *n*-hexyl, *n*-octyl, *n*-decyl, *n*-dodecyl, *iso*-propyl, *iso*-butyl, *tert*-butyl, 2,2-dimethylpropyl or cyclohexyl,
and wherein R3 comprises or consists of an aromatic 6-ring which contains one or two nitrogen atoms in its 6-ring, wherein the carbon atoms of the aromatic 6-ring can be substituted independently by H, or a C₁- to C₁₂-alkyl, e.g. methyl, ethyl, *n*-propyl, *n*-butyl, *n-*pentyl, *n*-hexyl, *n*-octyl, *n*-decyl, *n*-dodecyl, *iso*-propyl, *iso*-butyl, *tert*-butyl, 2,2-dimethylpropyl or cyclohexyl, preferably H or ethyl, or by a C₁- to C₁₂-alkoxyl of the aforementioned C₁- to C₁₂-alkyl groups, e.g. methoxyl, ethoxyl, or two neighbouring carbon atoms of the aromatic 6-ring can be substituted by at least one anellated phenyl ring, or by a halogen atom, preferably C1.

In the aromatic 6-ring of R3, in the embodiment of the 6-ring containing one nitrogen atom, or in the embodiment of the 6-ring containing two nitrogen atoms, the one nitrogen atom or one of the two nitrogen atoms can be in para-position, in ortho-position, or in meta-position to the carbonyl group of compound I, and in the embodiment of the 6-ring containing two nitrogen atoms, the other nitrogen atom can be directly neighbouring or with a spacing of one intermediate carbon atom between the two nitrogen atoms, e.g. one nitrogen atom in meta position, and the other nitrogen atom in ortho position or in para position, or one nitrogen atom in para position and the other nitrogen atom in ortho position.

Preferably, R3 is selected from the group A comprising

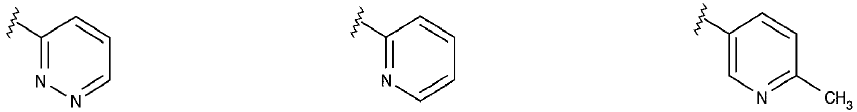

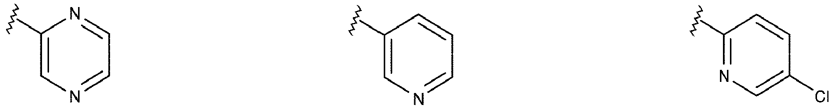

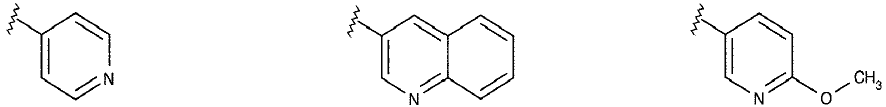

Among the R3 of this group A, the preferred nitrogen-containing aromatic 6-ring substituents comprise or consist of pyridine, wherein more preferably the nitrogen atom of the 6-ring is in meta position to the carbonyl group.

More preferably, especially for R3 being selected from group A, compound I is 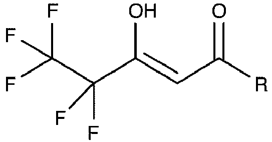 wherein the most preferred R3 is 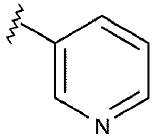 and wherein the carbon atoms of the nitrogen-containing aromatic 6-ring for which no substituent is explicitly indicated, are substituted with hydrogen, giving a compound corresponding to

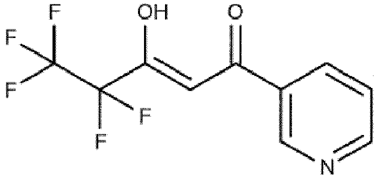

It was found that the nitrogen-containing aromatic 6-ring of R3 can be substituted with bulky substituents without significantly affecting the inhibition of FNT. Accordingly, the nitrogen-containing aromatic 6-ring of R3 can have further substituents, e.g. as also described above, the carbon atoms of the nitrogen-containing aromatic 6-ring for which no substituent is explicitly indicated, can be substituted with hydrogen or with a larger group, preferably in the 4-position of the nitrogen-containing aromatic 6-ring (para position to the carbonyl), e.g. a C₁- to C₁₂-alkyl, or a C₁- to C₁₂-alkoxyl, preferably a methoxy group in 4-position with the nitrogen of the 6-ring in the 2-position. A substituent to the nitrogen-containing aromatic 6-ring which is at least one anellated phenyl ring or a phenyl ring which is further substituted, is preferably bound to the carbon atoms in meta and para positions of the nitrogen-containing aromatic 6-ring. A phenyl ring which is further substituted can e.g. be substituted by a halogen atom, e.g. Cl, and/or a C₁- to C₁₂-alkyl or aryl as a further substituent.

When using cultivated Plasmodium falciparum, it was found that the active substances of the invention were also effective against a mutant that provided the FNT with some resistance against a comparative compound. Further, it was found that the presence of the active substances of the invention in cultivated Plasmodium falciparum did not result in the generation of resistance, indicating that the active substances of the invention do not result in eliciting a significant resistance in Plasmodium falciparum over the observed duration of cultivation of two weeks.

It was found that the active substances comprising or consisting of compound I affect the L-lactate transporter (FNT) of Plasmodium, resulting in the reduction of excretion of lactate, and hence in the death of Plasmodium due to acidification. The FNT of Plasmodium has no counterpart in humans, and therefore no prohibitive side effect of a treatment of humans using the active substances or of prophylactic administration of the active substances to humans is expected.

When using 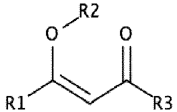 as a comparative compound with R1 being pentafluorethyl, R2 being H and R3 being a phenyl group substituted by an ethoxy group in para-position to the keto group, in cultivated Plasmodium falciparum after an initial high effectivity against Plasmodium, after some generations using lower concentrations of the compound, a mutation Gly107Ser in the FNT of P. falciparum was found that conferred some resistance.

The active substance compound of the invention has the advantage of being effective both against Plasmodium carrying the wild-type FNT, and against Plasmodium carrying the Gly107Ser mutant PfFNT. For the wild-type FNT of P. falciparum, compounds of the invention that have a pyridine containing R3 have been found to be highly effective against the wild-type FNT of P. falciparum at IC50 values of 0.15 µM or below. Exemplary compounds carrying an R3 with two nitrogen atoms showed a slightly weaker inhibition of the wild-type FNT of P. falciparum of 0.46 µM and 0.86 µM.

Exemplary wild-type FNT molecules are those of Plasmodium falciparum (Pf; PfFNT, SEQ ID NO: 1, Sequence-ID PF3D7_0316600 PlasmoDB), of Plasmodium vivax (Pv; PvFNT, Sequence-ID PVX_095405 PlasmoDB), of Toxoplasma gondii (Tg; TgFNT1, SEQ ID NO: 2, Sequence-ID TGGT1_209800 ToxoDB; TgFNT2, Sequence-ID TGGT1_292110 ToxoDB, SEQ ID NO: 3; TgFNT3, Sequence-ID TGGT1_229170 ToxoDB, SEQ ID NO: 4), the causative agent of toxoplasmosis, and of Entamoeba histolytica (Eh; EhFNT, Sequence-ID EHI_198990 AmoebaDB, SEQ ID NO: 5). For Naegleria fowleri (Nf), the amino acid sequences for FNT is known from ATCC 30894 tig74, as NfFNT1, whole genome shotgun, Sequence_ID: VFQX01000074.1, GenBank (SEQ ID NO: 6), as NfFNT2 whole genome shotgun, Sequence_ID: VFQX01000001.1, GenBank (SEQ ID NO: 7), and as a partial sequence NfFNT3, whole genome shotgun, Sequence_ID: VFQX01000009.1, GenBank (SEQ ID NO: 8). For Plasmodium vivax (Pv) the FNT has amino acid SEQ ID NO: 9, for Plasmodium knowlesi (Pk) the FNT has amino acid SEQ ID NO: 10, for Plasmodium ovale (Po) the FNT has amino acid SEQ ID NO: 11, and for Plasmodium malariae (Pm) the FNT has amino acid SEQ ID NO: 12.

The invention is now described by way of examples.

### Example: Inhibition of Plasmodium falciparum in culture

Plasmodium falciparum were kept in culture in 5% 0+ erythrocytes in RPMI 1640 medium containing 0,5% Albumax at 37 °C. Generally, IC50 was determined after 48 h following addition of test compounds by counting parasitemia using FACS (LSR II FACS, BD Biosciences). Medium containing test compounds was exchanged every 24 h. In order to test for the propensity of parasites to develop resistance to a given substance, 3D7 parasites were grown in the presence of 3 x IC₅₀ of a test for 30 days. Thereafter the drug was removed by exchanging the medium, and the day recorded when parasites resurfaced in the culture. As an alternative to Plasmodium falciparum, the FNT of the wild-type plasmodium (available under accession number PF3D7_0316600 in the data bank PlasmoDB) and the FNT (G107S mutant FNT) of the resistant plasmodia were expressed in the yeast Saccharomyces cerevisiae W303-1A jen1Δ ady2Δ from plasmid pDR196 (PMA promoter). In the yeast, the endogenous genes for the monocarboxylate transporter Jen1p (sequence accession No. CAA82062 NCBI) and Ady2p (sequence accession No. KZV12856) were deleted (obtained from M. Casal, Universidade do Minho, Portugal).

This yeast was grown in SD medium with addition of adenine, histidine, leucine, tryptophan and 2 % (wt/v) glucose at 30 °C up to an OD₆₀₀ of 0.8 to 1.0, harvested by centrifugation and washed once with water and centrifuged down again, and suspended in 50 mM HEPES/TRIS (pH 6.8) and adjusted to an OD₆₀₀ of 50± 10% and stored on ice. For contacting with one of the compounds a compound of one step of a dilution series in DMSO was provided in a reaction vessel and onto this 80 µL of the yeast suspension were pipetted. After an incubation on ice for 15 to 20 min 20 µL 5 mM Na-L-lactate plus 0.04 µCi radioactively labelled L-(1-¹⁴C) lactate was added. The lactate concentration obtained was 1 mM. After an incubation of 30 s the uptake of lactate was stopped by a rapid dilution by means of adding of 1 ml ice cold water. From the yeast suspension diluted this way the yeast cells were brought onto a filter membrane by vacuum filtration, washed with 7 ml cold water and transferred with the filter membrane into 3 ml scintillation cocktail. After 24 h of incubation at 18 °C in the scintillation cocktail, in which the cells were lysed, the amount of radioactively labelled lactate was measured by means of a scintillation counter. The total amount of lactate which was taken up by the yeast was calculated from the measured amount of radioactively labelled lactate. As a positive control the yeast was treated equally in parallel testing with DMSO without one of the compounds and was regarded as 100% FNT activity, respectively 0% inhibition. In tests with the compound a lower amount of lactate taken up was found in comparison to the parallel positive control. As a negative control yeast was treated in parallel in which the endogenous genes for the monocarboxylate transporters Jen1p and Ady2p were deleted, but the FNT from plasmodium was not expressed. This negative control was regarded as 0% FNT activity, respectively 100% inhibition.

The IC50 values determined using the yeast for the wild-type FNT and for the FNT mutant showed a similar shift as the IC50 values of the plasmodium culture. On the one hand, this shows that the tested compounds present their effect onto plasmodium by interaction with the FNT, respectively that FNT is the target molecule of these compounds in the inhibition of plasmodium. On the other hand, this result shows that the yeast expressing the FNT from plasmodium can be used as a representative for plasmodium itself in processes for analysis of the inhibitory effect of compounds onto plasmodium.

In the following table, IC50 values that were determined for comparative compounds are given:
IC50 values of comparative compounds against PfFNT

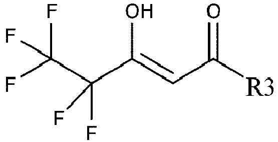

| | R3 | PfFNT wildtype IC₅₀ (µM) | PfFNT G107S IC₅₀ (µM) |
|---|---|---|---|
| compound [**1**] | 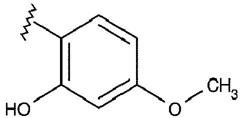 | 0.17±0.01 | 21±1 |
| compound [**2**] | 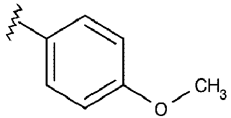 | 0.14±0.01 | 2.7±0.2 |
| compound [**3**] | 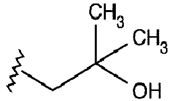 | 1.1±0.1 | 490±20 |
| compound [**4**] | 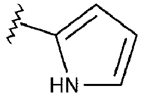 | 0.16±0.01 | 12±1 |
| compound [**5**] | 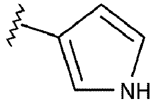 | 0.20±0.01 | 13±1 |
| compound [**6**] | 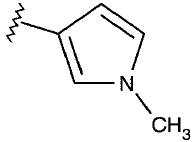 | 0.13±0.01 | 14±1 |
| compound [**7**] | 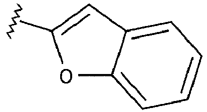 | 0.19±0.01 | 17±2 |

In the following table, IC50 values are given that were determined for preferred compounds according to the invention:
IC50 values of active substances of the invention against PfFNT

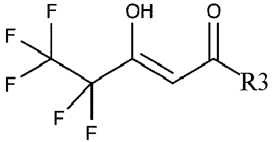

| | R3 | PfFNT wildtype IC₅₀ (µM) | PfFNT G107S IC₅₀ (µM) |
|---|---|---|---|
| compound [**12**] | 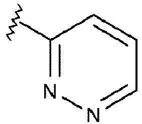 | 0.86±0.03 | 3.8±0.8 |
| compound [**13**] | 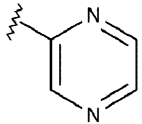 | 0.46±0.03 | 1.2±0.3 |
| compound [**14**] | 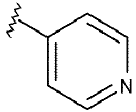 | 0.15±0.03 | 22 ± 7 |
| compound [**15**] | 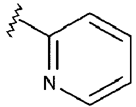 | 0.15±0.01 | 0.58±0.09 |
| compound [**16**] | 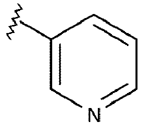 | 0.11±0.01 | 0.63±0.05 |
| compound [**17**] | 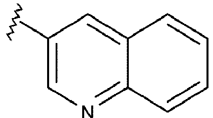 | 0.24±0.04 | 96±0.8 |
| compound [**18**] | 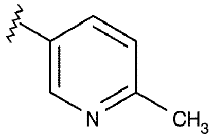 | 0.15±0.01 | 1.7±0.3 |
| compound [**19**] | 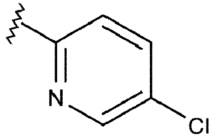 | 0.23±0.07 | 0.51±0.09 |
| compound [**20**] | 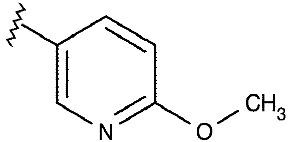 | 0.11±0.01 | 0.26±0.05 |
| compound [**21**] | 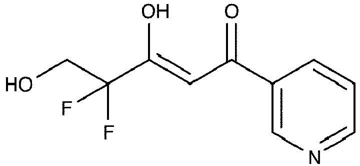 | 5.5 | n.d. |
| compound [**22**] | 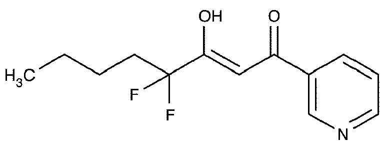 | 0.36±0.05 | 24±6 |

In R3 of compound 12, one of the nitrogen atoms of the aromatic 6-ring is in meta position, and one of the nitrogen atoms is in ortho position. In R3 of compound 13, one of the nitrogen atoms of the aromatic 6-ring is in meta position, and one of the nitrogen atoms is in para position.

These results show that the compounds of the invention are suitable for use in humans as active substances in the treatment or prevention of infections by protozoa. The active substances have activity against protozoa having the wild-type FNT, and against protozoa having the mutant FNT.

Further, the direct comparison of compound 20 according to the invention with comparative compound 2 shows that the nitrogen-containing aromatic 6-ring of R3 gives a lower IC50 value, i.e. a much stronger activity, against the wild-type FNT and especially against the resistant G107S mutant of FNT.

For the FNT of P. vivax (PvFNT), for compound 16 an IC50 of 0.229 +/- 0.01 µM was determined, for comparative compound 2 an IC50 of 0.249 +/- 0.08 µM, and for comparative compound 1 an IC50 of 0.0690 +/- 0.003 µM.
For the FNT of P. knowlesi (PkFNT), for compound 16 an IC50 of 0.874 +/- 0.06 µM was determined, for comparative compound 2 an IC50 of 0.985 +/- 0.08 µM, and for comparative compound 1 an IC50 of 0.212 +/- 0.07 µM.

For the FNT of P. ovale (PoFNT), for compound 16 an IC50 of 0.213 +/- 0.03 µM was determined, for comparative compound 2 an IC50 of ca. 0.9 µM.

For the FNT of P. malariae (PmFNT), for compound 16 an IC50 of 0.214 +/- 0.02 µM was determined, for comparative compound 2 an IC50 of 0.391 +/- 0.05 µM.

### Example 2: Syntheses of compounds

Exemplary methods for syntheses of active substances of the invention as a general procedure use a Claisen-type condensation in anhydrous THF in the presence of lithium hydride as generally described by Golldack 2017, quoted above, with purification by silica gel chromatography and sublimation. For structure analysis and purity, mass spectrometry (LC-MC, Bruker Amazon SL) and nuclear magnetic resonance (Bruker Avance III 300) were measured.

4,4,5,5,5-pentafluoro-3-hydroxy-1-pyridazin-3-yl-pent-2-en-1-one [compound 12] was produced by applying the general procedure with ethylpentafluoropropanoate (0.94 g, 4.9 mmol) and 3-acetylpyridazine (0.50 g, 4.1 mmol). 1H NMR (400 MHz, CDC13): δ 9.38 (dd, 5J = 1.6, 4J = 5.0, 1H), 8.27 (dd, 5J = 1.6, 3J = 8.4, 1H), 7.75 (dd, 4J = 5.0, 3J = 8.4, 1H), 7.63 (s, 1H). 13C-NMR (75 MHz, 25 °C, [D6]-DMSO): δ/ppm = 181.8 (1C), 169.1 (1C), 155.0 (1C), 154.3 (1C), 129.6 (1C), 126.8 (1C), 119.9 (1C), 117.1 (1C), 95.9 (1C).

4,4,5,5,5-pentafluoro-3-hydroxy-1-pyrazin-2-yl-pent-2-en-1-one [compound 13] was produced by applying the general procedure with ethylpentafluoropropanoate (2.88 g, 15 mmol) and 2-acetylpyrazine (0.5 g, 3.3 mmol). Yield 0.60 g, 2.2 mmol, 18 %. 1H NMR (400 MHz, CDC13): δ/ppm = 9.34 (d, 5J= 1.5, 1H), 8.81 (d, 3J = 2.4, 1H), 8.71 (dd, 5J= 1.5,3J= 2.4, 1H), 7.32 (s, 1H). 13C-NMR (75 MHz, 25 °C, CDC13): δ/ppm = 182.7 (1C), 140.2 (1C), 145.2 (1C), 144.2 (1C), 144.1 (1C), 119.4 (1C), 116.6 (1C), 107.4 (1C), 95.4 (1C). MS (ESI negative): m/z (%): 557 (100) [2M-2H+Na]-, 267 (4) [M-H]-.

4,4,5,5,5-pentafluoro-3-hydroxy-1-(4-pyridyl)pent-2-en-1-one [14] was produced by applying the general procedure with ethylpentafluoropropanoate (2.88 g, 15 mmol) and 4-acetylpyridine (1.51 g, 12.5 mmol). Yield 1.1 g, 4.1 mmol, 33 %. 1H NMR (400 MHz, CDC13): δ 8.85 (dd, 4J = 1.6, 3J = 4.5, 2H), 7.75 (dd, 4J = 1.6, 3J = 4.5, 2H), 6.67 (s, 1H). 13C-NMR (75 MHz, 25 °C, CDC13): δ/ppm = 182,07; 153,38; 151,01 (2C); 139,57; 120,40; 119,35 (2 C); 105,28; 94,36; MS (ESI negative): m/z (%): 555 (100) [2M-2H+Na]-, 266 (2) [M-H]-.

4,4,5,5,5-pentafluoro-3-hydroxy-1-(2-pyridyl)pent-2-en-1-one [15] was produced by applying the general procedure with ethylpentafluoropropanoate (2.88 g, 15 mmol) and 2-acetylpyridine (1.51 g, 12.5 mmol). Yield 1.2 g, 4.5 mmol, 36 %. 1H NMR (400 MHz, CDC13): δ/ppm = 14.3 (br s, 1H), 8.73 (m, 1H), 8.19 (m, 1H), 7.94 (m, 1H), 7.6 (m, 1H), 7.27 (s, 1H). 13C-NMR (75 MHz, 25 °C, CDC13): δ/ppm = 185.2 (1C), 150.2 (1C), 149.5 (1C), 137.6 (1C), 128.2 (1C), 127.7 (1C), 123.1 (1C), 119.6 (1C), 116.7 (1C), 95.2 (1C). MS (ESI negative): m/z (%): 555 (100) [2M-2H+Na]-, 266 (7) [M-H]-. The partition coefficient log*P* was calculated to 2.2.

4,4,5,5,5-pentafluoro-3-hydroxy-1-(3-pyridyl)pent-2-en-1-one [16] was produced by applying the general procedure with ethylpentafluoropropanoate (2.88 g, 15 mmol) and 3-acetylpyridine (1.51 g, 12.5 mmol). Yield 1.7 g, 6.4 mmol, 51 %. 1H NMR (400 MHz, CDC13): δ/ppm = 9.17 (dd, 5J = 0.8, 4J = 2.1, 1H), 8.85 (dd, 5J = 1.7, 3J = 4.8, 1H), 8.24 (dt, 4J = 1.7, 4J = 2.1, 3J = 8.0, 1H), 7.50 (ddd, 5J = 0.8, 3J = 4.8, 3J = 8.0, 1H), 6.66 (s, 1H)). 13C-NMR (75 MHz, 25 °C, CDC13): δ/ppm = 183.6 (1C), 179.5 (1C), 154.3 (1C), 148.8 (1C), 128.7 (1C), 123.8 (1C), 119.4 (1C), 116.6 (1C), 107.3 (1C), 94.0 (1C) MS (ESI negative):m/z (%): 555 (100) [2M-2H+Na]-, 266 (3) [M-H]-. The partition coefficient log*P* was calculated to 1.6, which indicates a higher solubility in water than e.g. log*P* of active substance [15].

4,4,5,5,5-pentafluoro-3-hydroxy-1-(3-quinolyl)pent-2-en-1-one [17] was produced by applying the general procedure with ethylpentafluoropropanoate (1.35 g, 7.0 mmol) and 3-acetylquinoline (1.00 g, 5.8 mmol). Yield 1.20 g, 3.8 mmol, 65 %. 1H NMR (300 MHz, CDC13): δ 9.35 (d, 4J = 2.3, 1H), 8.82 (d, 4J = 2.3, 1H), 8.24 (d, 3J = 8.4, 1H), 7.99 (dd, 3J = 8.4, 5J= 0.9, 1H), 7.89 (m, 1H), 7.69 (m, 1H), 6.77 (s, 1H)). 13C-NMR (75 MHz, 25 °C, CDC13): δ/ppm = 182.9 (1C), 148.8 (1c), 146.8 (1C), 137.7 (1C), 133.3 (1C), 129.4 (1C), 128.7 (1C), 128.5 (1C), 126.9 (1C), 125.6 (1C), 119.4 (1C), 116.5 (1C), 94.1 (1C). MS (ESI negative):m/z (%): 655 (100) [2M-2H+Na]-, 316 (1) [M-H]-.

4,4,5,5,5-pentafluoro-3-hydroxy-1-(6-methyl-3-pyridyl)pent-2-en-1-one [18] was produced by applying the general procedure with ethylpentafluoropropanoate (0.85 g, 4.4 mmol) and 5-acetyl-2-methylpyridine (0.5 g, 3.7 mmol). Yield 0.72 g, 2.6 mmol, 69 %. 1H NMR (400 MHz, CDC13): δ 9.05 (d, 4J = 2.3, 1H), 8.12 (dd, 4J = 2.3, 3J = 8.2, 1H), 7.32 (d, 3J = 8.2, 1H), 6.62 (s, 1H), 2.67 (s, 3H). 13C-NMR (75 MHz, 25 °C, CDC13): δ/ppm = 184.2 (1C), 178.9 (1C), 164.6 (1C), 148.5 (1C), 135.1 (1C), 126.0 (1C), 123.5 (1C), 119.4 (1C), 116.6 (1C), 93.6 (1C), 24.9 (1C). MS (ESI negative):m/z (%): 583 (100) [2M-2H+Na]-, 280 (1) [M-H]-.

1-(5-chloropyridin-2-yl)-4,4,5,5,5-pentafluoro-3-hydroxypent-2-en-1-one [19] was produced by applying the general procedure with ethylpentafluoropropanoate (1.48 g, 7.7 mmol) and 2-acetyl-5-chloropyridine (1.0 g, 6.4 mmol). 1H NMR (300 MHz, CDC13): δ/ppm = 8.76 (dd, J =2.1, J= 4.6, 1H), 8.58 (dd, J = 2.1, J = 7.8, 1H), 7.54 (dd, J = 4.6, J= 7.8, 1H), 6.81 (s, 1H) 13C-NMR (75 MHz, 25 °C, CDC13): δ/ppm = 177.4 (1C), 160.0 (1C), 154.5 (1C), 153.23 (1C), 136.8 (1C), 123.4 (1C), 119.1 (1C), 116.6 (1C), 113.1 (1C), 108.28 (1C) MS (ESI negative): m/z (%): 300 (100) [M-H]-.

4,4,5,5,5-pentafluoro-3-hydroxy-1-(6-methoxy-3-pyridyl)pent-2-en-1-one [20] was produced by applying the general procedure with ethylpentafluoropropanoate (0.76 g, 4.0 mmol) and 5-acetyl-2-methoxypyridine (0.5 g, 3.3 mmol). Yield 0.40 g, 1.3 mmol, 41 %. 1H NMR (400 MHz, CDC13): δ/ppm = 8.86 (d, J = 2.5, 1H), 8. 10 (dd, J = 2.5, J = 8.9, 1H), 6.85 (d, J = 8.9, 1H), 6.55 (s, 1H), 4.03 (s, 3H). 13C-NMR (75 MHz, 25 °C, CDC13): δ/ppm = 184.8 (1C), 177.2 (1C), 167.6 (1C), 148.8 (1C), 137.4 (1C), 122.4 (1C), 199.5 (1C), 166.7 (1C), 111.6 (1C), 93.1 (1C), 54.3 (1C). MS (ESI negative): m/z (%): 615 (100) [2M-2H+Na]-, 296 (1) [M-H]-.

4,4-difluoro-3,5-dihydroxy-1-(3-pyridyl)pent-2-en-1-one [21] was produced by applying the general procedure with ethyl 2,2-difluoro-3-hydroxypropanoate (0.50 g, 3.2 mmol) and 3-acetylpyridin (0.33 g, 2.7 mmol). Yield 0.33 g, 2.7 mmol, 44 %. 1H NMR (400 MHz, CDC13): δ 9.22 (dd, 5J = 0,8, 4J = 1.7, 1H), 8.82 (dd, 4J = 1.7, 3J = 4.8, 1H), 8.42 (dt, 4J = 1.9, 3J = 8.3, 1H), 7.60 (ddd, 5J = 0.8, 3J = 4.8, 3J = 8.3, 1H), 6.94 (s, 1H), 5.82 (bs, 1H), 3.92 (t, 2J = 14.15, 2H) . 13C-NMR (75 MHz, 25 °C, CDC13): δ/ppm = 185.17 (1C), 154.40 (1C), 150.23 (1C), 149.05 (1C), 135.70 (1C), 129.37 (1C), 124.64 (1C), 119.53 (1C), 94.98 (1C), 39.92 (1C). MS (ESI negative):m/z (%): m/z (%): 479 (100) [2M-2H+Na]-, 228 (6) [M-H]-.

4,4-difluoro-3-hydroxy-1-(3-pyridyl)oct-2-en-1-one [22] was produced by applying the general procedure with ethyl 2,2-difluorohexanoate (0.50 g, 2.8 mmol) and 3-acetylpyridine (0.28 g, 2.3 mmol). Yield 0.37 g, 1.5 mmol, 63 %. 1H NMR (300 MHz, CDC13): δ 9.15 (d, 3J = 1.9, 1H), 8.80 (dd, 3J = 1.9, 4J = 4.9, 1H), 8.22 (dt, 3J = 1.9, 4J = 8.0, 1H), 7.45 (dd, 4J = 4.9, 4J= 8.0, 1H), 6.60 (s, 1H), 2.11 (m, 2H), 1.48 (m, 2H), 1.41 (sx, 4J= 7.2, 2H), 0.93 (t, 4J = 7.2, 3H). 13C-NMR (75 MHz, 25 °C, CDC13): δ/ppm = 183.1 (1C), 153.6 (1C), 148.6 (1C), 134.7 (1C), 129.5 (1C), 123.6 (1C), 120.9 (1C), 118.4 (1C), 92.8 (1C), 33.7 (1C), 23.6 (1C), 22.3 (1C), 13.7 (1C). MS (ESI negative): m/z (%): 531 (100) [2M-2H+Na]-, 254 (2) [M-H]-.

## Claims

1. Substance, **characterized by** comprising or consisting of compound I 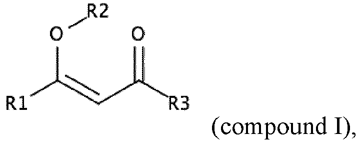
wherein R1 is perfluoro-Ci- to C₄-alkyl, straight chain or branched,
wherein R2 is H or a C₁- to C₁₂-alkyl, or R2 is a carbonyl group with H or a C₁- to C₁₂-alkyl,
and wherein R3 comprises an aromatic 6-ring which contains one or two nitrogen atoms in its 6-ring.

2. Substance according to claim 1, **characterized in that** the aromatic 6-ring of R3 contains a nitrogen atom in meta position to the carbonyl group.

3. Substance according to one of the preceding claims, **characterized in that** the aromatic 6-ring of R3 contains a nitrogen atom in ortho position to the carbonyl group.

4. Substance according one of the preceding claims, **characterized in that** a carbon atom of the aromatic 6-ring of R3 is directly linked to the carbon atom of the carbonyl group.

5. Substance according one of the preceding claims, **characterized in that** the aromatic 6-ring of R3 contains a nitrogen atom in para position to the carbonyl group.

6. Substance according to one of the preceding claims, **characterized in that** R1 is trifluoromethyl, pentafluoroethyl, heptafluoropropyl, or nonafluorobutyl, or a solvate or salt thereof.

7. Substance according to one of the preceding claims, **characterized in that** the C₁- to C₁₂-alkyl of R2 or the C₁- to C₁₂-alkyl of the carbonyl group of R2 is selected from methyl, ethyl, *n*-propyl, *n*-butyl, *n*-pentyl, *n*-hexyl, *n*-octyl, *n*-decyl, *n*-dodecyl, *iso-*propyl, *iso*-butyl, *tert*-butyl, 2,2-dimethylpropyl or cyclohexyl.

8. Substance according to one of the preceding claims, **characterized in that** at least one carbon atom of the aromatic 6-ring of R3 is substituted by H, or a C₁- to C₁₂-alkyl or by a C₁- to C₁₂-alkoxyl, and/or by a halogen atom.

9. Substance according to one of the preceding claims, **characterized in that** two carbon atoms of the aromatic 6-ring of R3 are substituted by at least one anellated aromatic 6-ring.

10. Substance according to one of the preceding claims, **characterized in that** R3 is selected from

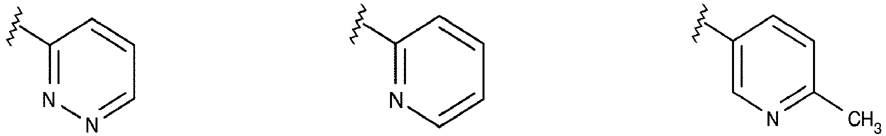

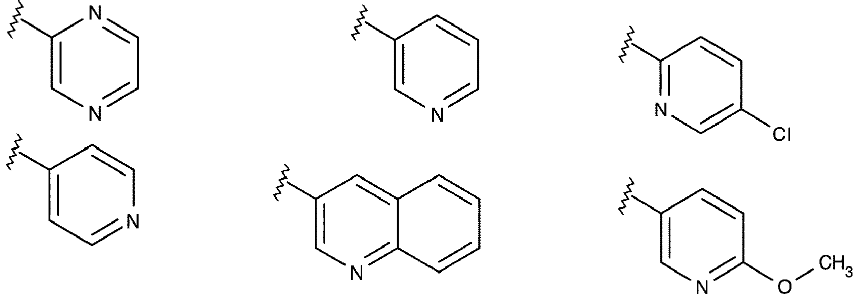

11. Substance according to one of the preceding claims, **characterized in that** the active substance is dissolved in an aqueous pharmaceutical formulation or is contained in a dry and water-soluble pharmaceutical formulation.

12. Substance according to one of the preceding claims for use in the treatment of and/or prevention of infection by protozoa.

13. Substance for use in the treatment and/or prevention of infection by protozoa according to claim 12, **characterized in that** the protozoon is Plasmodium spp. or Toxoplasma spp. or Entamoeba histolytica or Naegleria fowleri or Babesia bovis.

14. Substance for use in the treatment and/or prevention of infection by protozoa according to one of claims 12 to 13, **characterized in that** the protozoon has a FNT which has a G107S mutation in its FNT having SEQ ID NO: 1.
